# EUROPÄISCHE PATENTSCHRIFT

(11) **EP 1 331 944 B1**
(45) Veröffentlichungstag und Bekanntmachung des Hinweises auf die Patenterteilung: **20.05.2009**
(21) Anmeldenummer: 01986609.4
(22) Anmeldetag: 28.09.2001
(51) Int. Cl.: A61K 39/395, A61P 7/06, C07K 16/28

(54) **VERWENDUNG CD28 SPEZIFISCHER MONOKLONALER ANTIKÖRPER ZUR STIMULATION VON BLUTZELLEN, WELCHE KEIN CD28 TRAGEN**
USE OF CD28-SPECIFIC MONOCLONAL ANTIBODIES FOR STIMULATING BLOOD CELLS THAT LACK CD28
UTILISATION D'ANTICORPS MONOCLONAUX SPECIFIQUES A LA MOLECULE CD28 EN VUE DE STIMULER DES CELLULES SANGUINES QUI NE PORTENT AUCUNE MOLECULE CD28

(30) Priorität: 11.10.2000 DE 10050935
(43) Veröffentlichungstag der Anmeldung: 06.08.2003
(73) Patentinhaber: TheraMAB GmbH, 97076 Würzburg (DE)
(72) Erfinder: HÜNIG, Thomas, 97078 Würzburg (DE); RODRIGUEZ-PALMERO, Marta, 82377 Penzberg (DE); KERKAU, Thomas, 97070 Würzburg (DE)
(74) Vertreter: Jungblut, Bernhard Jakob
(86) Internationale Anmeldenummer: PCT/DE2001/003802
(87) Internationale Veröffentlichungsnummer: WO 2002/030459

(56) Entgegenhaltungen:
- WO-A-98/54225
- TACKE M ET AL: "CD28-mediated induction of proliferation in resting T cells in vitro and in vivo without engagement of the T cell receptor" EUROPEAN JOURNAL OF IMMUNOLOGY, WEINHEIM, DE, Bd. 27, Nr. 1, 1997, Seiten 239-247, XP002090176 ISSN: 0014-2980 in der Anmeldung erwähnt
- RODRIGUEZ-PALMERO ET AL: "Triggering of Tcell proliferation through CD28 induces GATA-3 and promotes T helper type 2 differentiation in vitro and in vivo" EUR. J. IMMUNOL, Bd. 29, - 1999 Seiten 3914-3924, XP001055132
- PEREZ BLAS M ET AL: "IMPAIRED T CELL SIGNAL TRANSDUCTION THROUGH CD28 IN A PATIENT WITH IDIOPATHIC THROMBOCYTOPENIA" CLINICAL AND EXPERIMENTAL IMMUNOLOGY, Bd. 85, Nr. 3, 1991, Seiten 424-428, XP001055131 ISSN: 0009-9104
- SIEFKEN RENATE ET AL: "CD28-mediated activation of resting human T cells without costimulation of the CD3/TCR complex." CELLULAR IMMUNOLOGY, Bd. 176, Nr. 1, 1997, Seiten 59-65, XP001027351 ISSN: 0008-8749 in der Anmeldung erwähnt
- BISCHOF ASTRID ET AL: "Autonomous induction of proliferation, JNK and NF-kappaB activation in primary resting T cells by mobilized CD28." EUROPEAN JOURNAL OF IMMUNOLOGY., Bd. 30, Nr. 3, März 2000 (2000-03), Seiten 876-882, XP001055187 ISSN: 0014-2980

## Beschreibung

### Gebiet der Erfindung

Die Erfindung betrifft eine Verwendung monoklonaler Antikörper, welche für CD28 spezifisch sind und T-Lymphozyten ohne Besetzung eines Antigenrezeptors der T-Lymphozyten und somit antigenunspezifisch aktivieren.

### Hintergrund der Erfindung

Es existieren verschiedene. Erkrankungen bei Warmblütlern, menschlich oder tierisch, wobei die Anzahl verschiedenartiger Blutzellen gegenüber dem Gesundzustand verringert ist.

Eine erste solche Gruppe von Erkrankungen wird Granulozytopenie (Neutropeniem und Monozytopenie) genannt und betrifft die Granulozyten. Als Ursachen für diese Erkrankung kommen in Frage: i) verminderte Granulozytopoese (aplastische Störung) aufgrund von Knochenmarksschädigung, beispielsweise durch Chemikalien, wie Benzol, Medikamente, wie Zytostatika, Immunsupressiva, AZT und/oder Chloramphenicol (dosisabhängig, toxisch) oder wie Phenylbutazon, Goldverbindungen, selten Chloramphenicol (dosisunabhängig durch pharmakogenetische Reaktionen), Strahlen oder Autoantikörper gegen Stammzellen (bei manchen Fällen von Immunneutropenie), aufgrund von Knochenmarksinfiltration (Leukämien, Karzinome, maligne Lymphome) und/öder aufgrund von Osteomyelosklerose, ii) Reifungsstörungen der Granulozytopoese, beispielsweise durch kongenitale Reifungsstörungen der Myelopoese, Kostmann-Syndrom (Reifungsstop der Myelopoese im Stadium des Promyelozyten), zyklische Neutropenie, Myelodysplasie-Syndrom, Vitamin B12- oder Folsäuremangel mit ineffektiver Granulo-, Erythro- und/oder Thrombopoese. Übliche Therapien umfassen die Gabe von Wachstumsfaktoren der Granulozytopoese (beispielsweise G-CSF und GM-CSF).

Eine zweite Gruppe wird Thrombozytopenie genannt. Als Ursachen kommen in Frage: i) verminderte Thrombozytopoese im Knochenmark (aplastische Störung bzw. verminderte Megakaryozytenzahl im Knochenmark) aufgrund von Knochenmarksschädigung, beispielsweise durch Chemikalien, wie Benzol, Medikamente, wie Zytostatika und/oder Immunsupressiva, Strahlen Infektionen, wie z.B. HIV, oder Autoantikörper gegen Megakaryozyten (bei manchen Fällen von Immunthrombozytopenie), aufgrund von Knochenmarksinfiltration (Leukämien, Karzinome, maligne Lymphome) und/oder aufgrund von Osteomyelosklerose, ii) Reifungsstörungen der Megakaryozyten (Megakaryozyten im Knochenmark normal oder erhöht) mit ineffektiver Thrombo- Erythro- und/oder Granulopoese mit Megaloblasten, Riesenstäben u.a. aufgrund von Vitamin B12-oder Folsäuremangel. Übliche Therapien umfassen das Weglassen verdächtiger Medikamente, Thrombozytensubstitution (bei Bildungsstörungen im Knochenmark: Thrombopoetin) sowie MGDF (Stimulation der Proliferation und Ausreifung von Megakaryozyten.

Eine dritte Gruppe bilden die aplastischen Anämien bzw. Knochenmarksversagen mit Aplasie/Hypoplaie des Knochenmarks und Panzytopenie (Stammzellenerkrankung). Eine angeborene aplastische Anämie ist beispielsweise die Fanconi-Anämie. Häufiger sind die erworbenen aplastischen Anämien, wie idiopatische aplastische Anämie (Ursache unbekannt) und sekundäre aplastische Anämien durch Medikamente, toxische Stoffe, ionisierende Strahlungen und Virusinfekte (s.o.). Supportive Therapieansätze umfassen die die Substitution von Erythrozyten/Thrombozyten. Kausale Therapieansätze umfassen die Knochenmarkstransplantation oder Stammzellentransplantation, Immunsuppressive Therapien (z.B. ATG) und andere Therapiemaßnahmen, wie Gabe von Zytokinen (GM-CSF, G-CSF, MGDF, und/oder Thrombopoetin).

Schließlich kommt die akute Leukämie, oft als Anämie, Thrombozytopenie und/oder Granulozytopenie, vor. Therapien umfassen die Substitution von Erythrozyten und Thrombozyten nach Bedarf bzw. die Anregung der Granulopoese durch G-CSF und/oder GM-CSF, die Chemotherapie und Knochenmarks- und/oder Stammzellentransplantation.

Den vorstehenden Erkrankungen ist gemeinsam, daß die betroffenen Blutzellen solche sind, die kein CD28 auf ihrer Oberfläche tragen.

### Stand der Technik

Als CD28 wird ein auf T-Lymphozyten menschlicher und tierischer Herkunft exprimiertes Zelloberflächenmolekül bekannter Aminosäuresequenz bezeichnet, dem im Rahmen der internationalen "Human Leukocyte Typing Workshops" das Kürzel CD28 gegeben wurde. Mit Aktivierung von T-Lymphozyten ist die Vermehrung der Stoffwechselaktivität, Vergrößerung des Zellvolumens, Synthese immunologisch wichtiger Moleküle und Eintritt in die Zellteilung (Proliferation) von T-Lymphozyten auf einen äußeren Reiz hin gemeint. Beispielsweise werden diese Vorgänge durch Besetzung des CD28-Moleküls auf T-Zellen durch besondere CD28-spezifische monoklonale Antikörper ausgelöst. Die Aktivierung von T-Lymphozyten mit den beschriebenen Begleiterscheinungen ist Teil der physiologischen Immunreaktion, kann dort aber in pathologischen Situationen außer Kontrolle geraten (lymphoproliferative Erkrankungen), oder unzureichend sein (Immundefizienz).

Zum Verständnis der Erfindung ist zunächst folgender technologischer Hintergrund wichtig. Die Aktivierung ruhender T-Zellen zur Proliferation und funktionellen Differenzierung erfordert zunächst die Besetzung zweier Oberflächenstrukturen, sogenannter Rezeptoren: 1. des Antigenrezeptors, der von Zelle zu Zelle eine unterschiedliche Spezifität besitzt und für die Erkennung von Antigenen, z. B. viralen Spaltprodukten, notwendig ist; sowie des auf allen ruhenden T-Zellen gleichermaßen exprimierten CD28 Moleküls, welches natürlicherweise an Liganden auf der Oberfläche anderer Zellen des Immunsystems bindet. Man spricht von der "Kostimulation" der antigenspezifischen Immunreaktion durch CD28. In Zellkultur können diese Vorgänge nachgestellt werden durch Besetzung des Antigenrezeptors sowie des CD28-Moleküls mit geeigneten monoklonalen Antikörpern. Im klassischen System der Kostimulation führt weder die Besetzung des Antigenrezeptors noch die des CD28-Moleküls allein zur T-Zellproliferation, die Besetzung beider Rezeptoren ist jedoch effektiv. Diese Beobachtung wurde an T-Zellen des Menschen, der Maus und der Ratte gemacht.

Eine "direkte", d. h. von der Besetzung des Antigenrezeptors unabhängige Aktivierung ruhender T-Lymphozyten durch CD28-spezifische monoklonale Antikörper, wurde in folgenden Systemen beobachtet: in der Literaturstelle Brinkmann et al., J. Immunology, 1996, 156: 4100-4106 wurde gezeigt, daß ein sehr kleiner Anteil (5 %) menschlicher T-Lymphozyten, die den für ruhende T-Lymphozyten typischen Oberflächenmarker CD45 RO tragen, durch den "klassischen" CD28-spezifischen monoklonalen Antikörper 9.3 bei Zusatz des Wachstumsfaktors Interleukin-2 (IL-2) ohne Besetzung des Antigenrezeptors aktiviert wird. In der Arbeit von Siefken et al., Cellular Immunology, 1997, 176: 59-65, wurde gezeigt, daß ein auf konventionellem Wege, d.h. durch Immunisierung von Mäusen mit menschlichen T-Zellen, hergestellter CD28-spezifischer monoklonaler Antikörper in Zellkultur eine Untergruppe menschlicher T-Zellen ohne Besetzung des Antigenrezeptors zur Proliferation aktivieren kann, wenn CD28 durch diesen monoklonalen Antikörper besetzt wird und die zellgebundenen monoklonale Antikörpermoleküle zusätzlich durch weitere Antikörper miteinander vernetzt werden. In beiden Fällen sind die beschriebenen Antikörper zunächst grundsätzlich nicht zum Einsatz in der Humanmedizin geeignet, da es sich um Maus Antikörper handelt. Weiterhin ist beiden beschriebenen Antikörpern gemeinsam, daß nur ein sehr kleiner Anteil der T-Zellen "direkt" aktivierbar ist.

In der Arbeit von Tacke et al., Eur. J. Immunol., 1997, 27:239-247 wurden zwei Arten von CD28-spezifischen monoklonalen Antikörpern mit unterschiedlichen funktionellen Eigenschaften beschrieben: "klassische Antikörper", die die Aktivierung ruhender T-Zellen nur bei gleichzeitiger Besetzung des Antigenrezeptors kostimulieren; und "direkte", die ohne Besetzung des Antigenrezeptors T-Lymphozyten aller Klassen in vitro und im Versuchstier zur Proliferation aktivieren können. Beide insofern bekannten monoklonale Antikörper rühren aus einer Immunisierung mit Zellen, auf denen Ratten-CD28 exprimiert ist, und sind durch auf ihre jeweiligen beschriebenen Eigenschaften gerichtete unterschiedlichen Selektionen erhältlich.

Aus der Literaturstelle WO 98/54225, auf welche zur Vermeidung von Wiederholungen ausdrücklich vollumfänglich Bezug genommen wird, sind "direkte" CD-28 spezifische monoklonale Antikörper bekannt sowie deren Verwendung zur Behandlung von Erkrankungen mit pathologisch erniedrigten CD4-T-Zellzahlen bzw. zur Modulation von Immunreaktionen bei Schutzimpfungen. Bei diesen Verwendungen ist regelmäßig auf Zellen abgestellt, welche CD28 auf ihrer Oberfläche tragen (T-Zellen), und folglich unmittelbar durch die monoklonalen Antikörper stimuliert werden.

Monoklonale anti-CD28 Antikörper, die T-Lymphozyten unspezifisch aktivieren, sind bekannt aus den Literaturstellen European Journal of Immunology, 27(1):239-247 (1997), European Journal of Immunology, 30(3):424-428 (2000), und Cellular Immunology 176(1):59-65 (1997). Aus der Literaturstelle Clinical and Experimental Immunology 85(3):424-428 (1991) ist es bekannt, dass ein an einer Thrombozytopenie erkrankter Patient ein CD-28 Defizit zeigte.

### Technisches Problem der Erfindung

Der Erfindung liegt das technische Problem zugrunde, eine pharmazeutische Zusammensetzung anzugeben, mittels welcher die Bildung und/oder Aktivierung von Blutzellen stimuliert wird, welche kein CD 28 tragen.

### Grundzüge der Erfindung

Zur Lösung dieses technischen Problems lehrt die Erfindung die Verwendung gemäß dem einzigen Patentanspruch.

Als Stimulation ist bezeichnet die Vermehrung der Stoffwechselaktivität, Vergrößerung des Zellvolumens, Synthese von Faktoren und/oder Eintritt in die Proliferation.

Analoge sind Substanzen, die keine monoklonalen Antikörper sind, jedoch die im Rahmen der Erfindung erläuterten Funktionen ausüben. Beispiele hierfür sind "geschneiderte" hochspezifische synthetische Proteine oder RNA, DNA oder PNA (z.B. Aptamere, insbesondere gegen Nukleinsäure-spaltende Enzyme stabilisierte Aptamere bzw. RNA, DNA oder PNA). Gemeinsames Kriterium ist stets die CD28 Spezifität mit "direkt" stimulatorischem Effekt auf CD28 tragende Blutzellen.

Die Erfindung beruht auf der überraschenden Erkenntnis, daß die CD28 spezifischen monoklonalen Antikörper auch die Proliferation bzw. Bildung bzw. Aktivierung von Blutzellen bewirken, die kein CD28 tragen, Blutzellen also, in denen eine Kopplung der Antikörper nicht möglich erscheint. Ohne an eine Theorie gebunden sein zu wollen, wird vermutet, daß die überraschende Wirkung darauf beruht, daß mittels der "direkten" Stimulation (also ohne ein Costimulans) von CD28 tragenden Zellen, insbesondere T-Zellen, diese Zellen ihrerseits Faktoren produzieren und abgeben, die dann die kein CD28 tragende Blutzellen wiederum stimulieren. Insofern liegt vermutlich ein indirekter Prozeß vor.

Bei pharmazeutischen Zusammensetzungen zur Gabe an Menschen weisen die erfindungsgemäß eingesetzten monoklonalen Antikörper vorzugsweise humane konstante Komponenten auf. Konstante Komponenten eines Antikörpers sind Bereiche, die nicht für die Antigenerkennung bedeutsam sind, im Gegensatz zu den variablen Bereichen, die die Antigenspezifität eines Antikörpers definieren. Konstante Komponenten unterscheiden sich jedoch bei Antikörpern verschiedener Arten und folglich auch Tieren und Menschen. Die konstanten Bereiche eines Antikörpers können beispielsweise jenen von Antikörpern eines Organismus entsprechen, der mit den Antikörpern behandelt werden soll, um verträglich zu sein. Erfindungsgemäß beim Menschen eingesetzte monoklonales Antikörper sind daher einerseits humanverträglich, sei es per se oder durch Humanisierung und können andererseits zur Behandlung dienen, da die Antikörper gegen Human-CD28 spezifisch ausgebildet sein können und da die Aktivierung der T-Lymphozyten umfassend ist. Mit entsprechender Anpassung bzw. Herstellung eines Derivats können erfindungsgemäß eingesetzte monoklonale Antikörper auch bei nicht-menschlichen Säugetieren eingesetzt werden. Es mag hier angemerkt sein, daß nicht humanisierte monoklonale Antikörper durchaus humanverträglich sein können. Humanverträglich sind monoklonale Antikörper daher dann, wenn sie über einen bestimmten Zeitraum keine unerwünschten Immunreaktionen auslösen, wie beispielsweise durch Bestimmung von anti-Immunglobulin Antikörper nachweisbar, wenn diese ihren Einsatz verbieten.

Unter Derivaten von monoklonalen Antikörpern sind Modifikationen des monoklonalen Antikörpers zu verstehen, die durch übliche biochemische oder gentechnische Manipulationen erzeugt wurden. Dies ist beispielsweise gegeben mit der Humanisierung eines monoklonale Antikörpers der Maus durch partiellen Ersatz struktureller (konstanter) Komponenten des Maus-Antikörpers durch solche eines menschlichen.

Im einzelnen sind erfindungsgemäß eingesetzte monoklonale Antikörper erhältlich durch: A) Herstellung von zur Produktion von monoklonalen Human-CD28 spezifischen Tier-Antikörpern befähigten Hybridomzellen im Wege einer Immunisierung mit nicht-T-Tumorzelllinien, auf welchen Human-CD28 exprimiert ist, oder mit rekombinant exprimiertem CD28, B) ggf. Humanisierung der aus Hybridomzellen gemäß Stufe A erhältlichen monoklonalen Tier-Antikörper durch biochemischen oder gentechnologischen Austausch konstanter Komponenten der Tierantikörper gegen analoge konstante Komponenten eines menschlichen Antikörpers bzw. Austausch den Komponenten entsprechender Gene der Hybridomzellen, C) Sezernierung der Antikörper in Hybridomzell-Kulturen und Isolierung der Antikörper daraus oder Produktion der Antikörper durch Injektion der Hybridomzellen in Tiere, beispielsweise Mäuse, und Isolierung der Antikörper aus der Körperflüssigkeit der Tiere. - Ein wichtiger Aspekt der erfindungsgemäß eingesetzten monoklonalen Antikörper besteht gegenüber den nächstliegenden Literaturstellen Brinkmann et al., J. Immunology, 1996, 156: 4100-4106, und Siefken et al., Cellular Immunology, 1997, 176: 59-65, demnach in der Erkenntnis, daß die monoklonalen Antikörper durch Immunisierung mit nicht-T Tumorzellen, auf welchen Human-CD28 exprimiert ist, erhalten sind, anstelle einer Immunisierung mit T-Zellinien. Denn so können monoklonale Antikörper erhalten werden, die nicht nur gegen (Human-) CD28 spezifisch sind, sondern auch eine "direkte" Aktivierung in beachtlichem Umfang bewirken. Im einzelnen können erfindungsgemäß eingesetzte monoklonale Antikörper Spezifität für Determinanten beispielsweise des menschlichen CD28-Moleküls aufweisen, die auf dem natürlicherweise exprimierten CD28-Molekül schwer zugänglich sind und deren Besetzung durch die neuartigen monoklonale Antikörper zur Aktivierung der T-Zellen und letztendlich zur Aktivierung von kein CD28 tragenden Blutzellen führt. Unter einer Determinante ist der Bereich eines Moleküls zu verstehen, der durch die Bindungsspezifität eines oder mehrerer Antikörper definiert wird.

Die grundsätzliche Vorgehensweise bei der Herstellung von Hybridomzellen, bei der Humanisierung sowie bei der Produktion der monoklonalen Antikörper aus (humanisierten) Hybridomzellen ist dem Fachmann gut vertraut und braucht hier nicht näher erläutert zu werden. Grundsätzlich sind alle insbesondere für die Herstellung der Hybridomzellen üblichen, bekannten und frei verfügbaren Zellinien einsetzbar. Zur Herstellung der monoklonalen Antikörper kommt grundsätzlich neben der folgend beschriebenen Vorgehensweise die dem Fachmann im Detail gut geläufige rekombinante Expression in Frage.

Im einzelnen ist es bevorzugt, wenn die zur Produktion von monoklonalen Human-CD28 spezifischen Tier-Antikörper befähigten Hybridomzellen erhältlich sind durch a) Schaffung eines Plasmids mittels Insertion von Human-CD28 cDNA in den pHßAPr-1-neo Vector nach Excision des SalI-HindIII Fragments und Herstellung von Protoplasten aus Escherichia coli (MC1061), welche das Plasmid tragen, b) Fusionierung der Protoplasten mit Maus A20J und/oder L929 Tumorzellen mittels Polyethylenglykol, c) Kultivierung der in Stufe b erhaltenen transfektierten Zellen, d) Screenen der transfektierten Maus A20J und/oder L929 Zellen auf die Expression von Human CD28 und Selektion von Human-CD28 exprimierenden Maus A20J und/oder L929 Zellen, e) Immunisierung von BALB/c Mäusen mit den Human-CD28 exprimierenden Maus A20J und/oder L929 Zellen (beispielsweise durch Injektionen 6 x i.p. und anschließend 1 x i.v.), f) Entnahme von Milzzellen der so immunisierten Mäuse und Fusionierung der Milzzellen mit ("nonproducer"-, d.h. keine Antikörper produzierenden) Zellen der Zellinie X63-Ag 8.653 mittels Polyethylenglykol, g) Selektierung der so erhaltenen Hybridomzellen mit der Maßgabe, daß im Überstand selektierter Hybridomzellen Antikörper enthalten sind, die an Human CD28 exprimierende Maus A20J und/oder L929 Zellen binden und h) Kultivierung/Subclonierung der in Stufe g erhaltenen selektierten Hybridomzellen. Anstelle der Stufen a) bis d) können selbstverständlich aber auch andere dem Fachmann geläufige Expressionsysteme eingesetzt werden. Human-CD28 cDNA ist frei erhältlich von Dr. A. Aruffo und Dr. B. Seed, die die Sequenz und auch folgende Literaturstelle veröffentlicht haben: Aruffo, A., and Seed, B, 1987, "Molecular cloning of a CD28 cDNA by a high efficiency COS cell expression system", Proc. Natl. Acad. Sci. USA, 84:8573. Dieser Literaturstelle ist daher im einzelnen die Herstellung der Human-CD28 cDNA entnehmbar. Darüberhinaus kann unschwer jeder Fachmann mit Hilfe der in der Genbank deponierten Sequenz und der Polymerasekettenreaktion sehr einfach und schnell einen Human-CD28 cDNA Klon herstellen. Der pHßAPr-1-neo Vector ist frei erhältlich von den Authoren der Literaturstelle Gunning, P, et al., 1987, "A human ß-actin expression vector system directs high-level accumulation of antisense transcripts", Proc. Natl. Acad. Sci. USA, 84:4831. "neo" steht dabei für Neomycin-Resistenz. Die Stufe c) wird daher in Anwesenheit von Neomycin durchgeführt. Die vorstehend angesprochenen Zellinien und/oder Mikroorganismen sind frei verfügbar und käuflich erwerbbar bei der American Type Culture Collection (ATCC). Bezüglich Escherichia coli (MC1061) wird ergänzend auf die Literaturstelle Meissner, P.S., et al., 1987, "Bacteriophage gamma cloning system for the construction of directional cDNA libraries", Proc. Natl. Acad. Sci. USA, 84:4171, verwiesen.

Die galenische Herrichtung der erfindungsgemäß eingesetzten monoklonalen Antikörper für die verschiedenen Verabreichungsformen ist dem Fachmann gut bekannt und braucht hier nicht näher erläutert zu werden. Insbesondere können neben der erfindungsgemäßen Wirkstoffkomponente noch weitere Wirkstoffe und/oder für die galenische Herrichtung zweckmäßige oder notwendige Stoffe enthalten sein. Es versteht sich, daß eine erfindungsgemäße pharmazeutische Zusammensetzung die monoklonalen Antikörper in einer therapeutisch wirksamen Dosis enthält. Eine therapeutisch aktive Dosis läßt sich für die verschiedensten Organismen dadurch ermitteln, daß die Dosis bestimmt wird, die statistisch signifikant zu einer Erhöhung der Anzahl an kein CD28 tragenden Blutzellen im Vergleich zu vor der Gabe der Zusammensetzung führt.

Die Erfindung umfaßt ein Verfahren zur Herstellung eines Medikamentes zur Heilung der einleitend genannten Krankheiten unter Verwendung erfindungsgemäßer monoklonaler Antikörper.

Im folgenden wird die Erfindung anhand von Ausführungsbeispielen näher erläutert.

Die dargestellten Experimente bzw. die Beispiele zu den Wirkungen von "direkten" CD28-spezifischen monoklonalen Antikörpern wurden im Tiermodell der Ratte durchgeführt, wobei als Beispiel für einen "klassischen" CD28-spezifischen Antikörper der monoklonale Antikörper JJ319 und als Beispiel für einen "direkt" aktivierenden der monoklonale Antikörper JJ316 eingesetzt wird. Beide Antikörper sind frei verfügbar und käuflich erwerbbar von der Firma Pharmingen, San Diego, USA. JJ319 und JJ316 Antikörper sind im übrigen erhältlich gemäß der Literaturstelle M. Tacke et al., Immunology, 1995, 154: 5121-5127, auf welche hiermit ausdrücklich Bezug genommen wird, auch im Hinblick auf Details der Herstellung von Hybridomzellen und monoklonalen Antikörpern.

### Beispiel 1: Herstellung CD28 spezifischer "direkter" monoklonaler Antikörper.

In diesem Beispiel wird die Herstellung erfindungsgemäß eingesetzter, d.h. Human-CD28-spezifischer monoklonaler Antikörper näher erläutert. Diese werden folgend auch als CMY-2 bezeichnet. Human CD28 aus einer cDNA Bibliothek wurde in A20J und/oder L929 Zellinien rekombinant exprimiert. Zunächst wurde hierzu ein Plasmid mittels Insertion von Human-CD28 cDNA in den pHßAPr-1-neo Vector nach Excision des SalI-HindIII Fragments geschaffen. Aus Escherichia coli (MC1061) wurden Protoplasten hergestellt, welche das Plasmid tragen. Dann erfolgte eine Fusionierung der Protoplasten mit Maus A20J und/oder L929 Tumorzellen mittels Polyethylenglykol. Die so erhaltenen transfektierten Zellen wurden auf übliche Weise kultiviert. Anschließend erfolgte ein Screenen der transfektierten Maus A20J und/oder L929 Zellen auf die Expression von Human-CD28 und Selektion von Human-CD28 exprimierenden Maus A20J und/oder L929 Zellen.

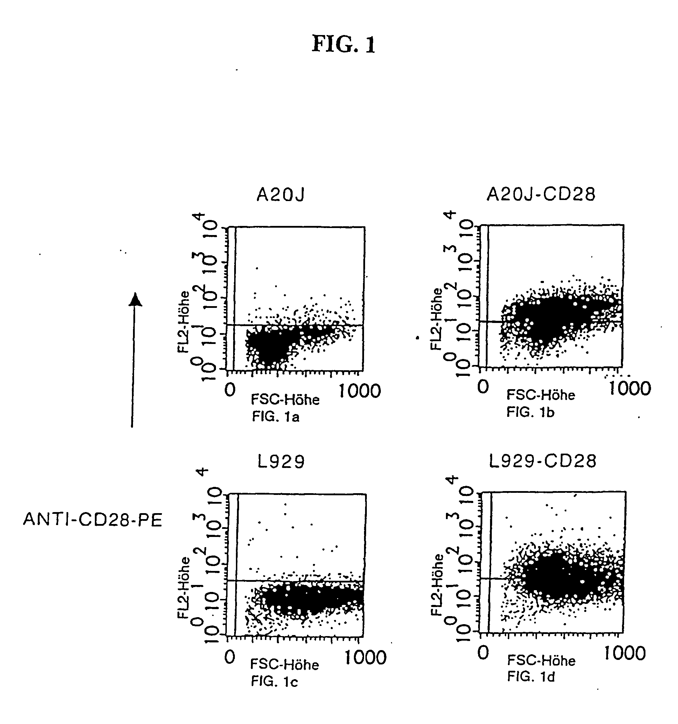

Der Nachweis der erfolgreichen Expression erfolgte mit Hilfe eines konventionellen, kommerziell erhältlichen fluoreszenzmarkierten Antikörpers mit Spezifität für Human CD28 (9.3-Phykoerythrin). Als Negativkontrolle wurden nicht transfizierte A20J- bzw. L929-Zellen mit dem gleichen Antikörper gefärbt. Die Transfektanten (A20J-CD28 und L929-CD28) zeigten eine höhere Fluoreszenzintensität. Da nicht alle Zellen CD28 positiv waren, wurden CD28-positive Zellen subkloniert und zur Immunisierung verwendet. Wie in Fig. 1 an der Verschiebung der Punktwolken nach oben in den beiden rechten Diagrammen erkennbar, reagierten diese Zellen mit dem käuflichen Antikörper, drückten also Human CD28 an ihrer Oberfläche aus.

Die A20J Human-CD28 Zellinie wurde zur Immunisierung von BALB/c Mäusen verwendet. Zellfusion und screening wurden wie folgt durchgeführt: i) Immunisierung von BALB/c Mäusen mit den Human-CD28 exprimierenden Maus A20J Zellen (Injektionen 6 x i.p. und anschließend 1 x i.v.). ii) Entnahme von Milzzellen der so immunisierten Mäuse und Fusionierung der Milzzellen mit Zellen der Zellinie X63-Ag 8.653 mittels Polyethylenglykol. iii) Selektierung der so erhaltenen Hybridomzellen mit der Maßgabe, daß im Überstand selektierter Hybridomzellen Antikörper enthalten sind, die an Human-CD28 exprimierende Maus A20J und/oder L929 Zellen binden.

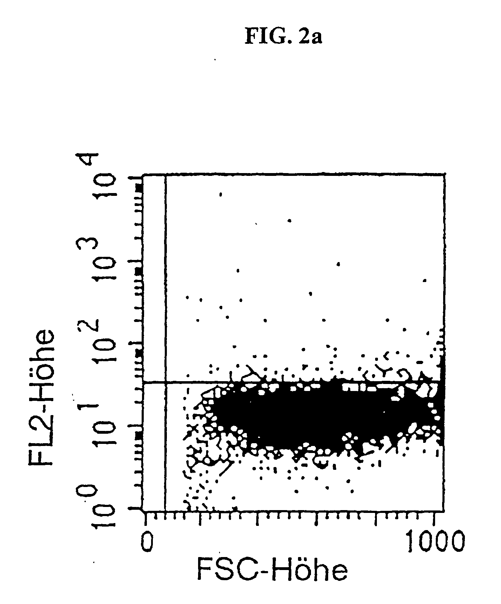

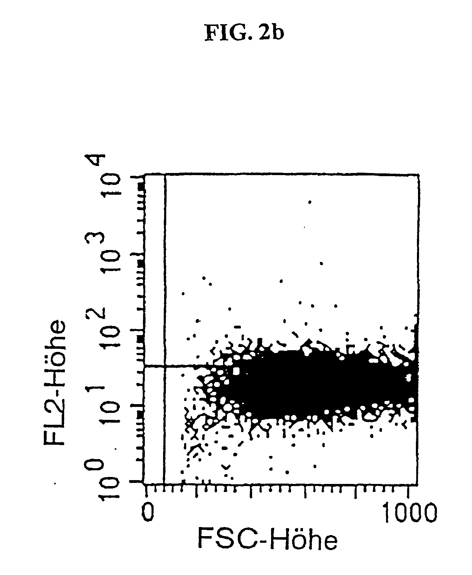

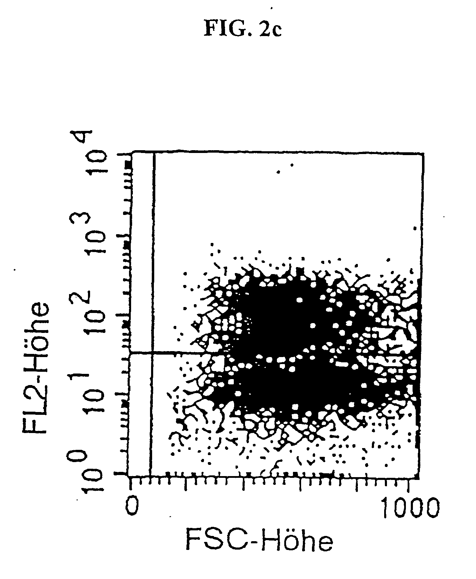

Als read-out diente die Anfärbung einer Mischung aus CD28 transfizierten und untransfizierten Maus L929 Tumorzellen. Fig. 2 zeigt, daß der auf diesem Weg isolierte monoklonale Antikörper CMY-2 transfizierte und untransfizierte Zellen durch unterschiedliche Fluoreszenzintensität unterscheidet. Das differentielle Screening auf Antikörper gegen Human-CD28 erfolgte wie folgt. Je 50 µl Überstand von kultivierten Zellhybridomen wurden entnommen und mit einem Gemisch aus L929-Zellen und L929-CD28-Transfektanten 15 min inkubiert. Nach dem Waschen wurden die Zellen mit DaMIg-PE angefärbt. Teil A zeigt die Negativkontrolle. Die Zellen wurden nur mit DaMIg-PE inkubiert. Teil B zeigt die Färbung mit einem Überstand, der leicht positiv war, aber keinen Unterschied bei beiden Zellen zeigt. Teil C zeigt die mit einem Überstand von CMY-2 gefärbten Zellen.

In nicht dargestellten Experimenten wurden periphere Blutzellen des Menschen mit dem neu isolierten CMY-2 und dem "klassischen" CD28-spezifischen Antikörper 9.3 gefärbt. Es wurde ein identisches Expressionsmuster auf den Subpopulationen menschlicher Blutzellen gefunden.

Zusammengefaßt zeigen die Experimente, daß CMY-2 ein Human CD28-spezifischer Antikörper ist.

CMY-2 wurde sodann mit aus peripherem Blut auf circa 80 % angereicherten menschlichen T-Lymphozyten auf klassische kostimulierende und auf "direkt" stimulierende Aktivität getestet. Die T-Zellproliferation wurde durch Einbau von ³H-Thymidin zwischen dem 2. und 3. Tag der Kultur gemessen. Folgende Ergebnisse wurden erzielt:

**Kostimulation:**

| | |
|---|---|
| Unstimulierte Zellen | 276 cpm |
| CD3-spezifischer Antikörper | 3111 cpm |
| CD3-spezifischer Antikörper + CMY-2 | 51676 cpm |

**Direkte Stimulation:**

| | |
|---|---|
| Solid-phase anti-mouse Ig | 379 cpm |
| Solid-phase anti-mouse Ig + Kontroll-mAk | 258 cpm |
| Solid-phase anti-mouse Ig plus CMY-2 | 19115 cpm |

Zur Erläuterung: Anti-CD3 sorgt für T-Zellrezeptor-Stimulation (CD3 ist Teil des TCR-Komplexes). CMY-2 wurde in Form eines nicht aufgereinigten Kulturüberstandes (50% Endvolumen) verwendet. Erfahrungsgemäß ist die dabei zu erwartende effektive mAk-Konzentration suboptimal für eine direkte Aktivierung, aber ausreichend für die Kostimulation. Das Experiment zeigt, daß CMY-2 direkt aktivierende Eigenschaften hat.

Erfindungsgemäße Hybridomzellen, welche CMY-2 produzieren, sind bei der DSMZ, Deutsche Sammlung von Mikroorganismen und Zellkulturen GmbH, Mascheroder Weg 1b, D-38124 Braunschweig, unter der Nummer DSM ACC2353 (20.05.1998) hinterlegt worden.

### Beispiel 3: Rekonstitution der T-Zellenzahl in T-lymphopänischen Ratten mit mitogenen anti-CD28-mAk.

Behandlung mit mitogenen nicht aber mit konventionellen anti-CD28 MAK führt zu rascher Rekonstitution der T-Zell zahlen in T-lymphopänischen Ratten, wie folgend gezeigt werden wird.

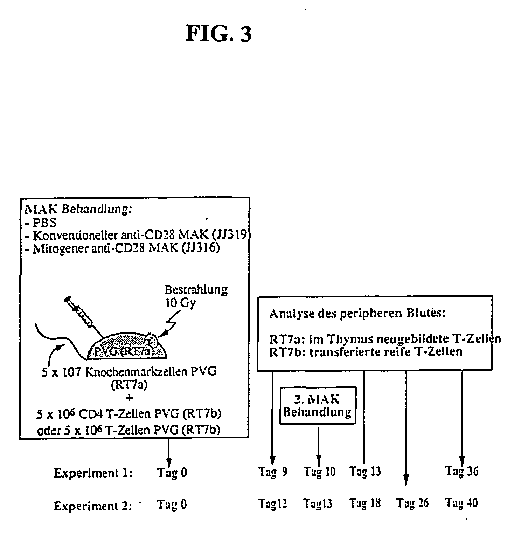

Der Versuchsplan ist in Fig. 3 dargestellt. PVG Inzuchtratten wurden mit Gammastrahlung aus einer Cäsiumquelle so bestrahlt, daß das blutbildende System einschließlich der weissen Blutzellen zerstört wird. Als Quelle blutbildender Stammzellen erhielten die Tiere Knochenmarkzellen des gleichen Inzuchtstamms i.v. Zusätzlich erhielten sie in Experiment 1 5x10⁶ CD4 T-Zellen und in Experiment 2 5x10⁶ T-Zellen (CD4 und CD8) des kongenen Inzuchtstamms PVG.RT7^{b}. Diese Tiere sind bis auf das Leukozytenoberflächenmolekül RT7, von dem sie das Allel b statt a exprimieren, mit dem Empfängerstamm PVG identisch. Mit Hilfe RT7^{b}-spezifischer fluoreszenzmarkierter MAK können so in den Tieren T-Zellen identifiziert werden, die von den 5x10⁶ injizierten reifen T-Zellen abstammen. Solche T-Lymphozyten, die aus den unreifen Vorläuferzellen des injizierten Knochenmarks im Thymus der Empfängertiere neu zu T-Lymphozyten reifen, reagieren hingegen nicht mit RT7^{b}-spezifischen fluoreszenzmarkierten MAK. Die Zahl von 5x10⁶ T-Lymphozyten entspricht ungefähr einem tausendstel der Zahl an reifen T-Lymphozyten einer adulten Ratte, so daß ein Modell drastischer T-Lymphopenie vorliegt. Zur Vermehrung der injizierten T-Zellen im Empfängertier wurde entsprechend dem gezeigten Behandlungsschema 1 mg des mitogenen anti-CD28 MAK JJ316 i.v. an zwei Tagen (Tag 0 und Tag 10 in Expt. 1, Tag 0 und Tag 13 in Expt. 2) appliziert. Zu den angegebenen Zeitpunkten wurde den Tieren Blut abgenommen, und die Zahl der T-Lymphozyten wurde durch Messung der Gesamtzahl der Leukozyten sowie des Anteils an T-Lymphozyten daran bestimmt. Weiterhin wurde unterschieden zwischen T-Lymphozyten, die von den reifen injizierten T-Zellen abstammen (RT7^{b} positiv) und denen, die im Thymus neu gebildet wurden (RT7^{b} negativ). Diese Analysen wurden mit Hilfe monoklonaler Antikörper mit Spezifität für T-Zelloberflächenmarker durchgeführt. Die verwendete Technik ist die Mehrfarbenimmunfluoreszenz in Verbindung mit der Durchflusszytophotometrie ("FACS^{®}"-Analyse).

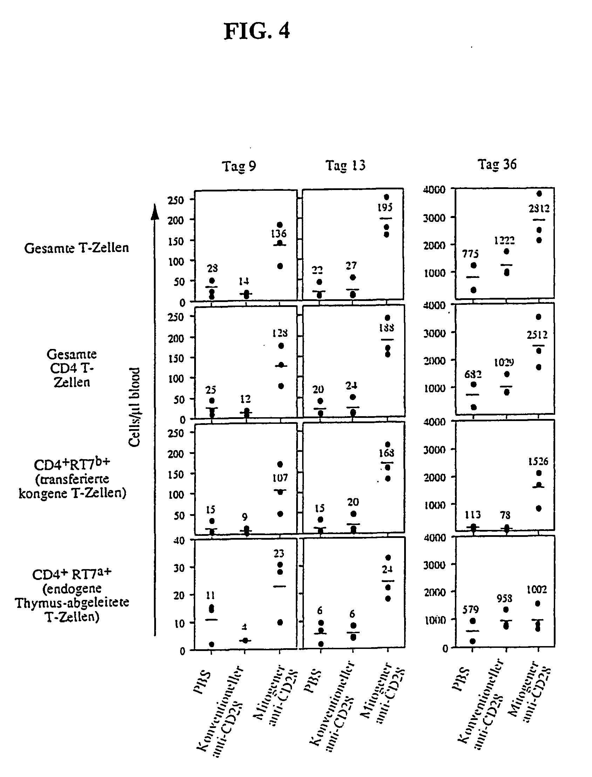

In der Figur 4 ist das Experiment 1 dargestellt, nämlich Rekonstitution mit reifen CD4 T-Zellen. Die verschiedenen Behandlungen der Tiere sind auf der Abszisse vermerkt; jedes Tier wird durch einen Punkt symbolisiert. Bereits neun Tage nach Versuchsbeginn zeigte sich ein deutlicher Vorsprung in der Zahl von T-Lymphozyten im peripheren Blut in der mit mitogenen anti-CD28 MAK behandelten Gruppe gegen über Kontrolltieren, die konventionellen anti-CD28 MAK oder nur die Phosphat-gepufferte Kochsalzlösung (PBS) erhalten hatten, die als Lösungsmittel für die MAK verwendet worden war. Dieser Vorsprung blieb bis zum letzten Messpunkt (Tag 36) erhalten. Werden ausschließlich die von den reifen RT7^{b}-positiven CD4 T-Zellen abstammenden T-Lymphozyten betrachtet, fällt der therapeutische Effekt des mitogenen anti-CD28 MAK noch dramatischer gegenüber den Kontrollgruppen aus. Der Grund dafür liegt darin, daß mit fortschreitender Zeit im Thymus neue, RT7^{b} -negative T-Zellen gebildet werden. Die Entstehung dieser T-Zellen ist unabhängig von der Antikörperstimulation, wurde durch diese jedoch auch nicht beeinträchtigt.

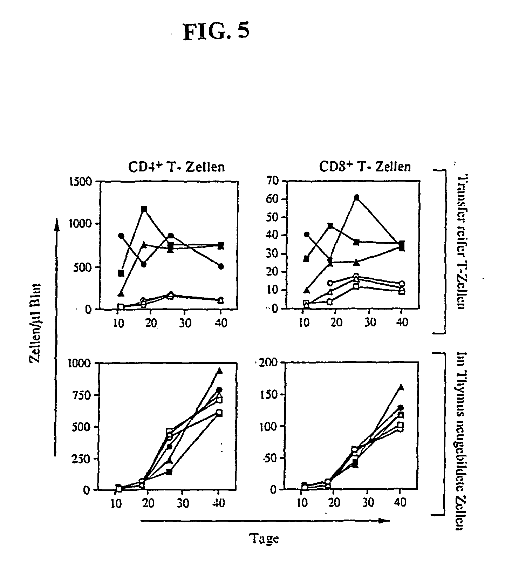

Die Figur 5 zeigt das Experiment 2, nämlich Rekonstitution mit reifen CD4 und CD8 T-Zellen. Entsprechend dem Schema in Fig. 3 wurden 5x10⁶ RT7^{b}-positive T-Lymphozyten transferiert; diese bestehen zu etwa 3/4 aus CD4- und zu etwa 1/4 aus CD8 T-Zellen. Die T-Zellzahlen im Blut wurden wie zur Figur 5 beschrieben bestimmt. Jede Linie repräsentiert ein Versuchstier. Offene Symbole zeigen die Behandlung mit PBS an, geschlossene die mit dem mitogenen anti-CD28 MAK JJ316. Die oberen beiden Graphen zeigen die Entwicklung der Zahlen von RT7^{b}-positiven Zellen an, die sich von dem reifen T-Zellinokulum ableiten; die unteren beiden die der RT7^{b}-negativen, die im Thymus neu gebildet wurden. Die Ergebnisse zeigen eine rasche Vermehrung der reifen CD4 und CD8 T-Lymphozyten durch die Behandlung mit mitogenem anti-CD28 MAK (oben). Die später einsetzende Neubildung von T-Zellen (unten) wurde durch die Behandlung nicht beeinträchtigt.

### Beispiel 4: Qualität der vermehrten T-Lyphozyten

In diesem Beispiel wird gezeigt, daß T-Lymphozyten, die durch anti-CD28 Therapie vermehrt worden waren langlebig und funktionsfähig sind.

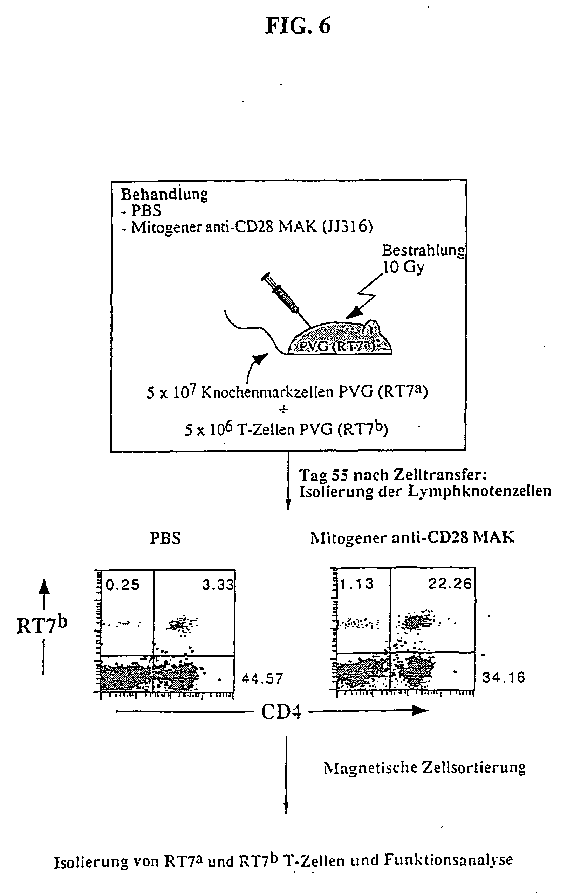

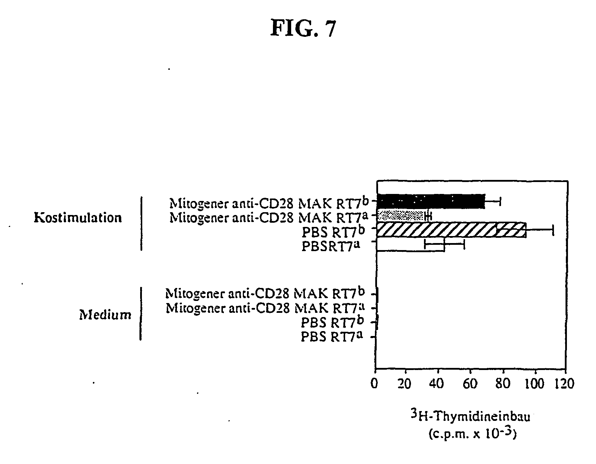

Die Figur 6 zeigt den Versuchsplan und Nachweis von durch anti-CD28 vermehrten T-Zellen in den Lymphknoten. Die Behandlung der Versuchstiere entspricht zunächst dem in Fig. 4, Experiment 2 erläuterten Schema. Nach 55 Tagen wurden Tiere getötet. Die Analyse der T-Lymphozyten in den Lymphknoten ergab, daß nach PBS Behandlung nur 6% (3.33 von 47.9%) sich von dem zu Versuchsbeginn injizierten Inokulum reifer RT7^{b}-positiver Zellen ableitete; die übrigen CD4 T-Zellen waren im Thymus neu gebildet worden (RT7^{b}-negativ). Im Gegensatz dazu betrugen die RT7^{b}-positiven CD4 T-Zellen in der therapierten Gruppe etwa 40% (22.26 von 56.42%). Die dargestellten "Dotplots" zeigen wiederum die Ergebnisse der in Beispiel 3 beschriebenen durchflusszytophotometrischen Analysen. Es wurden fiuoreszenzmarkierte Antikörper gegen CD4 (Abszisse) und RT7^{b} (Ordinate) verwendet. Dieses Resultat zeigt, daß die zu Versuchsbeginn (Tag 0 und Tag 13) durchgeführte Therapie zu einer lang anhaltenden Repopulation mit den durch die anti-CD28 Therapie vermehrten T-Lymphozyten führt. RT7^{b}-positive und RT7^{b}-negative T-Lymphozyten wurden voneinander getrennt. Dazu erfolgte zunächst eine Anreicherung der T-Zellen mit Hilfe der Passage über Nylonwolle. Sodann wurden RT7^{b}-positive T-Zellen mit RT7^{b}-spezifischen MAK beladen, an die paramagnetische Eisenkügelchen gebunden wurden. Mit Hilfe eines Magneten erfolgte dann die Trennung zwischen RT7^{b}-positiven und RT7^{b}-negativen T-Zellen. Das hier verwendete, in der Forschung weit verbreitete System der Firma Miltenyi Biotech, Bergisch-Gladbach, heißt MACS (Magnetism Activated Cell Sorting). Die so getrennten RT7^{b}-positiven und RT7^{b}-negativen T-Zellen wurden dan n einer Funktionsanalyse unterzogen (Figuren 7 - 9).

Figur 7 zeigt die in vitro Proliferation von in vivo therapeutisch expandierten T-Zellen. Die wie in Figur 6 beschrieben isolierten RT7^{b}-positiven,- also vom ursprünglichen reifen Inokulum abgeleiteten T-Zellen sowie die RT7^{b}-negativen ("RT7^{a}", neu im Thymus gebildeten) T-Zellen wurden in einem Standard-Proliferationstest auf ihre Reaktivität gegenüber konventioneller Kostimulation überprüft. Dazu wurden 1x10⁵ T-Zellen in 0,2 ml Kulturmedium in 96-Napf Mikrotiterplatten zwei Tage bei 37 °C kultiviert, gefolgt von einem 16-stündigen Puls mit 1 µCi³H-markierten Thymidins. Dessen Einbau in die DNA ist als cpm x 10⁻³ dargestellt und ist ein akzeptiertes Maß für die Zellteilungsaktivität (Proliferation) der Zellen. Die oberen vier Balken zeigen Ansätze, in denen die T-Zellen mit Hilfe eines T-Zellantigenrezeptor (TCR)-spezifischen MAK (R73) sowie zusätzlich mit dem konventionellen CD28-spezifischen MAKJJ319 stimuliert wurden (klassische Kostimulation). Hier wurden die physiologischen Signale bei der T-Zellaktivierung nachgestellt. Die vier Balken zeigen die Reaktion der vier verschiedenen getesteten T-Zellpopulationen, nämlich jeweils die aus dein ursprünglichen Inokulum reifer T-Zellen stammenden RT7^{b} sowie die im Thymus neu gebildeteten RT7^{a} Zellen aus den beiden Tiergruppen, nämlich den CD28-therapierten und den mit PBS behandelten Kontrolltieren. Die untere Gruppe ("Medium") zeigt die gleichen Zellen ohne Stimulation. Hier wurde keine Proliferation beobachtet. Der Versuch zeigt, daß auch nach der extensiven in vivo Expansion durch CD28-Therapie die Reaktionsfähigkeit der TLymphozyten erhalten bleibt.

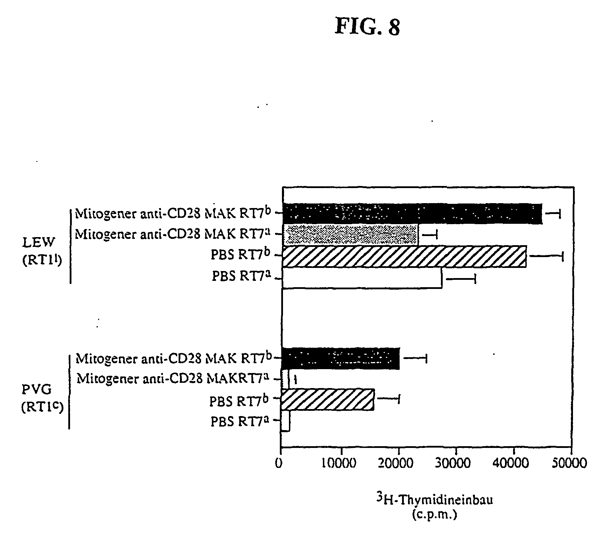

Figur 8 zeigt, daß in vivo durch anti-CD28 Therapie-vermehrte T-Zellen gegen fremde Transplantationsantigene reagieren. Der Versuchsansatz enspricht im Prinzip dem zur Figur 7 beschriebenen, nur daß die Stimulation der T-Zellen hier nicht mit Hilfe monoklonaler Antikörper durchgeführt wurde, sondern durch Zugabe sogenannter Stimulatorzellen des Stammes LEW. Dabei handelt es sich um 10⁵ bestrahlte Lymphknotenzellen pro Napf. Die Bestrahlung wird durchgeführt, damit die Stimulatorzellen selbst nicht zur gemessenen Proliferation beitragen können. Der Stamm LEW wurde gewählt, da er sich in seinen starken Transplantationsantigenen (entsprechend den HLA Antigenen des Menschen) von PVG unterscheiden: LEW exprimiert RT1¹, PVG hingegen RT1^{c}. Zur Ermittelung eines Basiswertes wurden in der unteren Gruppe auch bestrahlte PVG Lymphknotenzellen als Stimulatoren eingesetzt. Das Ergebnis zeigt, daß wiederum sämtliche der vier untersuchten Gruppen von T-Lymphozyten mit Proliferation auf die fremden Transplantationsantigene reagieren; darüber hinaus zeigt sich eine erhöhte Hinter- grundreaktion bei den T-Zellen sowohl der therapierten wie auch der Kontrollgruppe, die aus dem ursprünglich applizier- ten Inokulum reifer T-Zellen (RT7^{b}-positiv) abstammten.

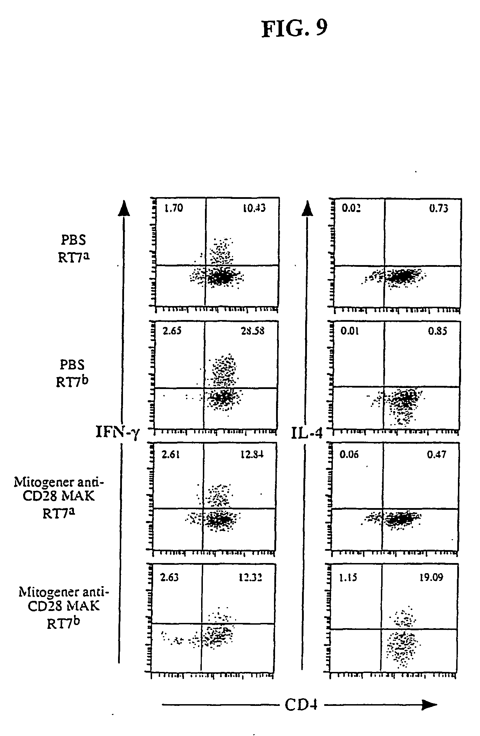

Die Figur 9 zeigt, daß in vivo durch anti-CD28 Therapie vermehrte T-Zellen in der Lage sind, Zytokine zu produzieren. Wie zur Figur 7 beschrieben, wurden die expandierten reifen (RT7^{b+}) und die im Thymus neu gereiften (RT7^{b-}, entspricht RT7^{a}) T-Zellen der anti-CD28 therapierten und der Kontrolltiere in vitro durch Kostimulation aktiviert. Nach 5 Tagen wurden die Zellen geerntet und nach einem Protokoll, das sich im Katalog der Firma Pharmingen/Becton Dickinson nachlesen lässt, auf die Fähigkeit zur Produktion der Zytokine gamma-Interferon und Interleukin-4 getestet. Diese beiden Zytokine wurden gewählt, weil sie charakteristisch sind für die beiden funktionell unterschiedlichen Typen von CD4 Effektor T-Zellen, die nach Aktivierung entstehen können: Entzündungsfördernde TH1 Zellen produzieren IFNgamma, entzündungshemmende und Antikörperproduktion fördernde TH2 Zellen produzieren IL-4. Es handelt sich bei der Nachweismethode um eine intrazelluläre Zytokinfärbung, die durchflusszytophotometrisch ausgewertet wird. Jeder Punkt oberhalb der horizontal verlaufenden Trennlinie zeigt eine Zelle an, die das an der Ordinate angegebene Zytokin synthetisiert. Es ist offensichtiich, daß sämtliche 4 Gruppen von T-Lymphozyten eine ähnlich große Fraktion von Zellen enthalten, die IFNgamma produzieren können. Im Gegensatz dazu finden sich IL-4 produzierende Zellen nur bei denjenigen T-Lymphozyten; die in vivo durch mitogene anti-CD28 Therapie vermehrt worden waren (Dotplot unten rechts). Das Experiment zeigt, daß durch die in vivo Expansion mit mitogenen CD28-spezifischen MAK die Fähigkeit, Zytokine vom TH1-Typ zu synthetisieren, nicht verloren geht. Das Auftreten von Zellen vom TH2 Phänotyp (W-4 produzierend) überrascht nicht.

### Beispiel 5: Vermehrung von T-Zellen durch CD28 Therapie in thymektomierten Ratten.

Der Versuchsansatz entspricht dem in Fig. 3 dargestellten mit zwei Abweichungen: 1. Die Tiere erhielten nur eine einmalige Injektion von 1 mg MAK am Tag 0; 2. Den Tieren wurde vor Bestrahlung und Rekonstitution operativ der Thymus entfernt, um das Nachreifen von T-Zellen im Thymus zu verhindern. Damit wird die Situation der Lymphopänie beim erwachsenen Menschen z. B. nach Chemo- oder Strahlentherapie nachgestellt. Erwachsene Menschen sind zur Neuproduktion von T-Zellen im Thymus kaum in der Lage, da nach der Pubertät die Thymusfunktion weitgehend eingestellt wird

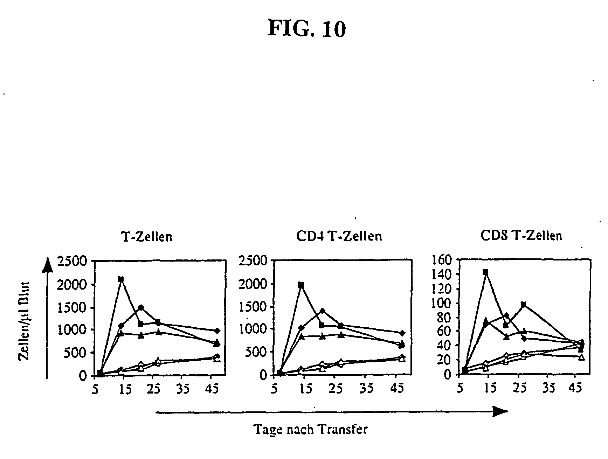

Die Figur 10 zeigt die Erholung der T-Zellzahlen im Blut. Eine Unterscheidung zwischen RT7^{b}-positiven und -negativen, Zellen ist nun nicht mehr notwendig, da sämtliche T-Zellen RT7^{b+} sind (fehlende Neuproduktion von RT7^{b-} T-Zellen im Thymus). Die drei Graphen zeigen die T-Zellen sowie ihre CD4 und CD8 Subpopulationen von jeweils drei anti-CD28 therapierten (ausgefüllte Symbole) und drei mit einem isotypgleichen Kontrollantikörper behandelten Tieren (offene Symbole). Wie sich leicht erkennen lässt, führte die einmalige Behandlung mit 1 mg des mitogenen anti-CD28 mAk JJ316 zu einer sehr raschen Erholung der T-Zellzahlen.

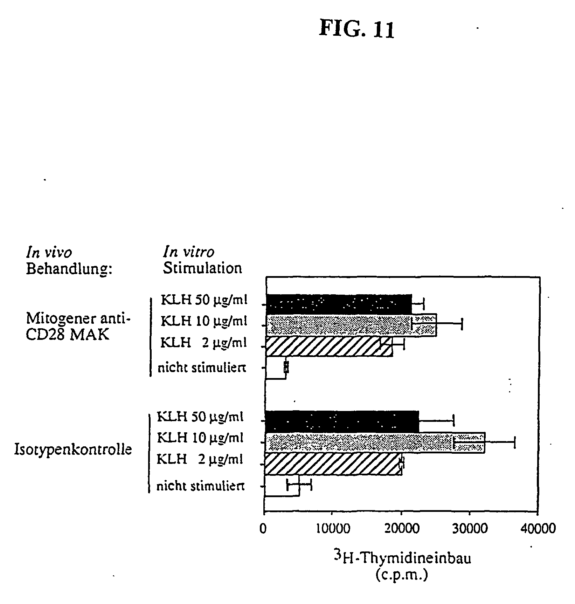

In der Figur 11 ist erkennbar, daß durch anti-CD28 Therapie in vivo vermehrte T-Zellen in vivo immunisiert werden können. Die zur Figur 10 beschriebenen Tiere wurden 4 Wochen nach Behandlung mit dem Modellantigen Keyhole Limpet Hämozyanin (KLH) immunisiert, um die Funktionsfähigkeit der T-Lymphozyten zu testen. Nach weiteren 4 Wochen wurden die Tiere getötet und die Lymphknotenzellen wurden in vitro auf ihre Fähigkeit getestet, auf KLH-Stimulation mit Proliferation zu reagieren. Man nennt dies den Test auf ein "Recall" Antigen; ein solcher Test wird z. B. beim Menschen angewandt, um die erfolgreiche Induktion einer T-Zellimmunität auf Impfantigene wie Tetanustoxoid zu testen. Das Testsystem entspricht grundsätzlich dem der Figuren 7 und 8, doch wurde zur Stimulation das Impfantigen KLH verwendet. Es ist offensichtlich, daß die Tiere beider Gruppen eine T-Zellimmunität gegen KLH in Folge der Impfung besaßen. Diese fällt in beiden Gruppen ähnlich stark aus, da für den in vitro Test die Zellzahlen pro Kulturnapf angeglichen werden, so daß Unterschiede in der vorausgegangenen therapeutischen T-Zellexpansion durch mitogene anti-CD28 mAk aufgehoben werden.

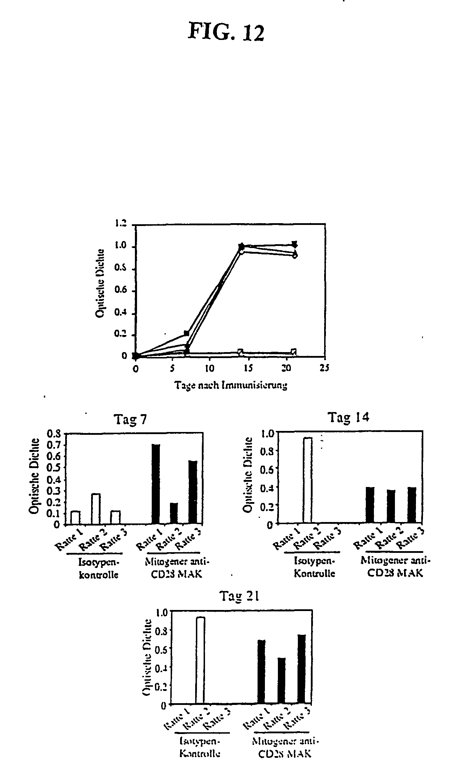

Figur 12 zeigt die Produktion von Antikörpern gegen das Modellantigen KLH. Die Produktion von Antikörpern durch B-Lymphozyten ist abhängig von der Funktion von CD4 T-Lymphozyten. Aus den in Figur 11 beschriebenen KLH-immunnsierten Tieren wurde deshalb zu den auf der Abzisse ersichtlichen Zeitpunkten Blut entnommen und in einem Enzyme-Linked ImmunoSorbent Assay (ELISA) auf Anwesenheit KLH-spezifischer Antikörper untersucht. Wie die obere Grafik zeigt, reagierten sämtliche anti-CD28-therapierten Tiere mit der gleichen raschen Kinetik auf die Immunisierung (gefüllte Symbole) während zwei der drei Kontrolltiere keine Reaktion zeigten; das dritte reagierte ähnlich wie die therapierte Gruppe, wenn auch mit verzögerter Kinetik (siehe Graphen der ELISA Ergebnisse der Tage 7, 14 und 21; diese Werte wurden in einem zweiten Test gemessen; die gemessene optische Dichte ist deshalb mit der des oberen Graphen nicht direkt vergleichbar).

In einem nicht durch eine Figur dargestellten Experiment wurde gezeigt, daß anti-CD28-therapierte T-lymphopenische Tiere friemde Hauttransplantate abstoßen. Die Bekämpfung von Virus-infizierten Zellen, von Tumoren und von intrazellulären Bakterien ist abhängig von der Funktion pro-inflammatorischer TH1 Zellen. Wegen der Anwesenheit anti-inflammatorischer IL-4 produzierender TH2 Zellen in CD28-therapierten T-lymphopenischen Tieren mußte die Möglichkeit in Betracht gezogen werden, daß die pro-inflammatorische, "zellvermittelte" Immunität unterdrückt wird. Dieses läßt sich anhand der Fähigkeit der Tiere prüfen, ein fremdes Hauttransplantat abzustoßen. Auch diese Reaktion ist TH1-abhängig. Die CD28-therapierten Tiere erhielten deshalb ein Hauttransplan tat des Stammes LEW, der die RT1¹ Allele der starken Transplantationsantigene exprimiert, während die therapierten PVG Tiere RT1^{c} tragen. Zur Kontrolle wurde ein syngenes PVG Transplantat übertragen. Sämtliche der insgesamt acht untersuchten therapierten Tiere stießen die fremde Haut ab, während die vom genetisch identischen Stamm einwuchs. Beispielhaft war einerseits das nekrotische LEW und andererseits das gut vaskularisierte PVG Transplantat bei einem Tier zu sehen. Im Durchschnitt war die Abstoßung durch die therapierten Tiere schneller als durch die Kontrolltiere. Dieses Ergebnis belegt, daß durch anti-CD28 Therapie die zeilvermittelte Immunität nicht verloren geht.

### Beispiel 6: Erhöhung der Granulozytenzahlen im Blut anti-CD28 mAk-behandelter Ratten.

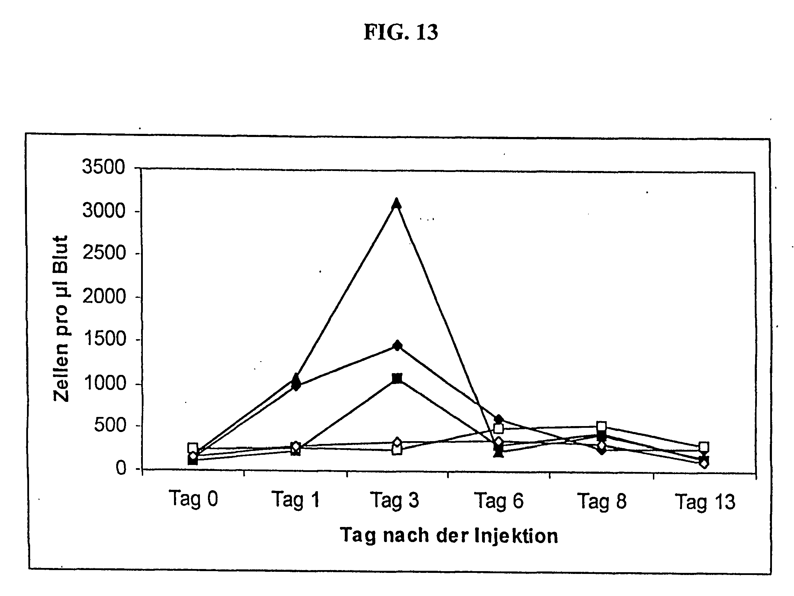

Die Fig. 13 zeigt eine Kinetik der Granulozytenzahlen nach Behandlung mit konventionellem (offene Symbole) und mit mitogenem anti-CD28 mAk (gefüllte Symbole). Jede Linie steht für ein Tier. Adulte LEW Ratten erhielten 1 mg des mitogenen anti-CD28 mAk JJ316 oder des konventionellen anti-CD28 mAk JJ319 i.v. appliziert. Die Zahl der Granulozyten im peripheren Blut wurde zu den gegebenen Zeitpunkten durch Bestimmung der Gesamtleukozytenzahl sowie des Anteils der Granulozyten daran bestimmt. Diese Messung erfolgte mit der Durchflusszytophotometrie auf Grund der besonderen Lichtstreuungseigenschaften von Granulozyten. Wie die Fig. 13 zeigt, wurde in allen Tieren, die mitogenen anti-CD28 mAk erhalten hatten, eine dramatische transiente Steigerung der Granulozytenzahlen beobachtet, nicht aber in den Kontrolltieren, die konventionellen anti-CD28 mAk erhalten hatten.

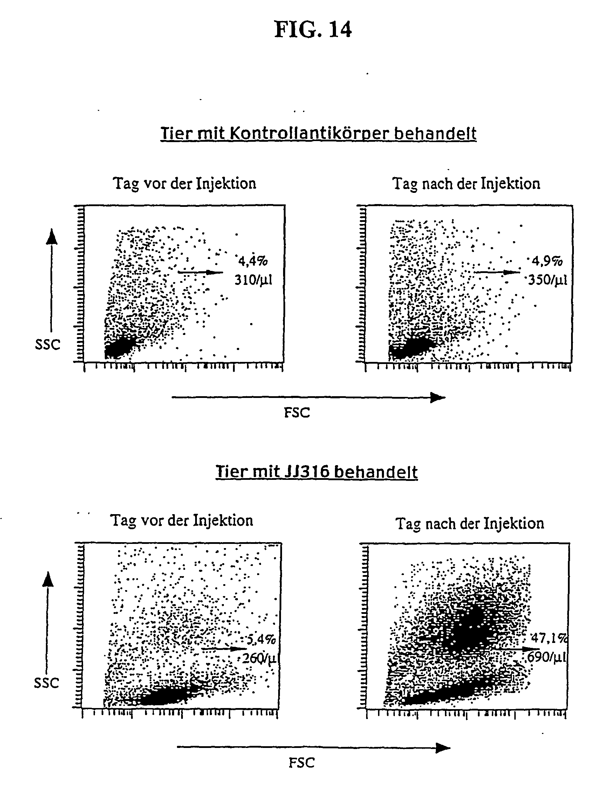

Die Fig. 14 zeigt ein Beispiel für die erhöhte Granulozytenzahl im Blut CD28-stimulierter Tiere. Die Leukozyten wurden in einem Durchflusszytophotometer auf ihre Lichtstreuungseigenschaften untersucht. FSC bedeutet forward scatter oder Lichtstreuung nach vorne, SSC sideward scatter oder die nach 90° abgelenkte Lichtstreuung, die ein Maß für die Granularität der Zellen ist. Jede Zelle wird durch einen Punkt dargestellt. Die beschriftete Wolke zeigt die Granulozyten an. Deren Prozentsatz ergibt sich direkt aus der Messung, die beigefügte Zellzahl pro Mikroliter Blut wird mit Hilfe der zuvor bestimmten Zahl von Leukozyten pro Mikroliter Blut errechnet. Man erkennt eine weit über die Signifikanzgrenze hinausgehende Erhöhung der Granulozytenzahl nach Behandlung mit "direkten" CD28 spezifischen mAk.

### Beispiel 7: Erhöhung der Monozytenzahlen nach Behandlung mit konventionellem und mit mitogenem anti-CD28 mAk.

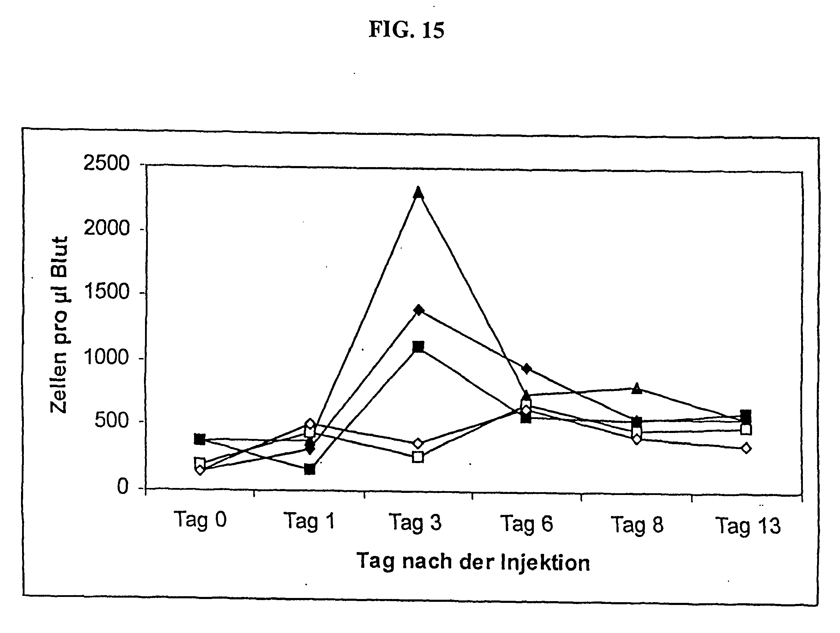

Adulte LEW Ratten erhielten 1 mg des mitogenen anti-CD28 mAk JJ316 (gefüllte Symbole) oder des konventionellen anti-CD28 mAk JJ319 (offene Symbole) i.v. appliziert. Die Zahl der Monozyten im peripheren Blut wurde zu den gegebenen Zeitpunkten durch Bestimmung der Gesamtleukozytenzahl sowie des Anteils der Monozyten daran bestimmt. Diese Messung erfolgte mit der Durchflusszytophotometrie auf Grund der besonderen Lichtstreuungseigenschaften von Monozyten sowie der Expression des Mac-1 Antigens, das durch den fluoreszenz-markierten MAK 0X42 nachgewiesen wurde. Wie die Fig. 15 zeigt, wurde in allen Tieren, die mitogenen anti-CD28 mAk erhalten hatten, eine dramatische transiente Steigerung der Monozytenzahlen beobachtet, nicht aber in den Kontrolltieren, die konventionellen anti-CD28 mAk erhalten hatten.

### Beispiel 8: Vermehrung von Granulozyten und Monozyten im Blut bestrahlter Knochenmark-rekonstituierter Ratten durch anti-CD28 Therapie.

Nach Beeinträchtigung des hämatopoetischen Systems z.B. durch Strahlen- oder Chemotherapie müssen auch die dem angeborenen oder natürlichen Immunsystem zugerechneten Granulozyten und Monozyten neu gebildet werden. Da aktivierte T-Lymphozyten diese z.B. durch Zytokinproduktion (G-CSF, GM-CSF) stimulieren können, und da in gesunden Tieren eine Erhöhung der Granulozyten- und Monozytenzahlen im Blut nach anti-CD28 Behandlung beobachtet worden war, wurde die Fähigkeit mitogener anti-CD28 MAK geprüft, die Erholung dieser Leukozytenpopulationen zu beschleunigen.

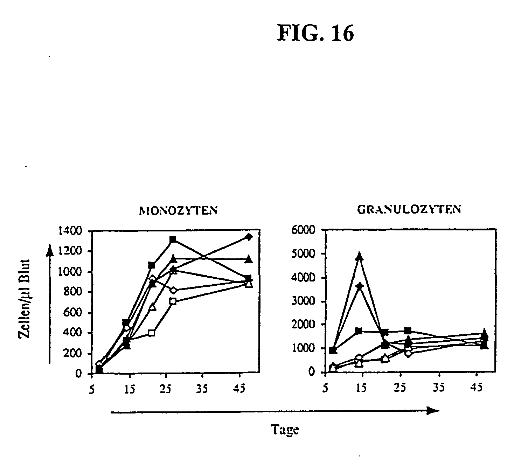

Fig. 16 zeigt den zeitlichen Verlauf von Granulozyten- und Monozytenzahlen im Blut bestrahlter Knochenmark-rekonstituierter Ratten nach anti-CD28 Therapie

PVG Inzuchtratten wurden zunächst operativ der Thymus entfernt, um das Nachreifen von T-Zellen zu verhindern (damit wird die Situation der Lymphopänie beim Menschen, beispielsweise nach Chemo- oder Strahlentherapie, nachgestellt; bei erwachsenen Menschen ist die Thymusfunktion weitgehend eingestellt). Sie wurden dann mit Gammastrahlung aus einer Cäsiumquelle so bestrahlt, daß das blutbildende System einschließlich der weissen Blutzellen zerstört wurde. Als Quelle blutbildender Stammzellen erhielten die Tiere Knochenmarkszellen des gleichen Inzuchtstammes i.v.. Zusätzlich erhielten sie 5 x 10⁶ CD4 T-Zellen oder 5 x 10⁵ T-Zellen (CD4 und CD8) des kongenen Inzuchtstammes PVG.RT7^{b}. Die Tiere dieses Stammes sind bis auf das Leukozytenoberflächenmolekül RT7, von dem sie das Allel b anstatt a exprimieren, mit dem Empfängerstamm PVG identisch. Mit Hilfe RT7b-spezifischer fluoreszenzmarkierter MAK können so in den Tieren T-Zellen identifiziert werden, die von den injizierten reifen T-Zellen abstammen. Die Anzahl der injizierten T-Zellen entspricht ungefähr einem Tausendstel der Zahl an reifen T-Zellen einer adulten Ratte, so daß ein Modell drastischer T-Lymphopenie vorliegt. Den Tieren wurden am Tag 0 1 mg JJ316 bzw. JJ319.

Zu den angegebenen Zeitpunkten wurde Blut abgenommen und die Zellzahlen entsprechend dem Beispiel 7 bestimmt. Der Fig. 16 entnimmt man eine schnellere Erholung der Monozyten- und Granulozytenzahl mit den erfindungsgemäß eingesetzten mAk, wobei im Falle der Granulozyten in den ersten Tagen nach Behandlung eine überschießende Reaktion gefunden wird, die sich dann wieder normalisiert.

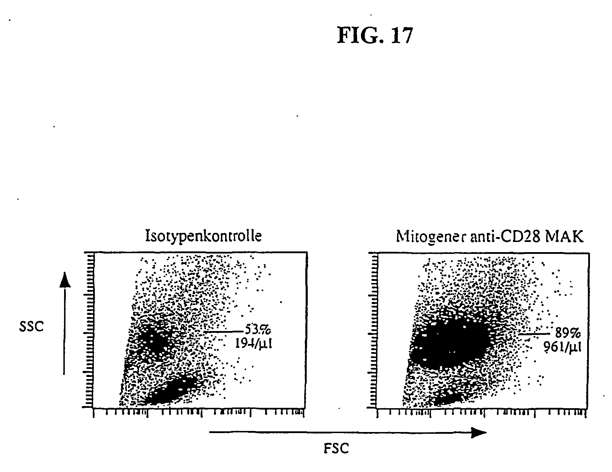

Die Fig. 17 zeigt Daten zum Anteil an Granulozyten im Blut bestrahlter Knochenmark-rekonstituierter Ratten nach anti-CD28 Therapie. Als Beispiel ist eine Messung des Granulozytenanteils 7 Tage nach Beginn der Therapie gezeigt. Die Methodik entspricht der in Beispiel 3 beschriebenen.

### Beispiel 9: Stimulation in Rhesusaffen mit einem mitogenen CD28-spezifischen Antikörper.

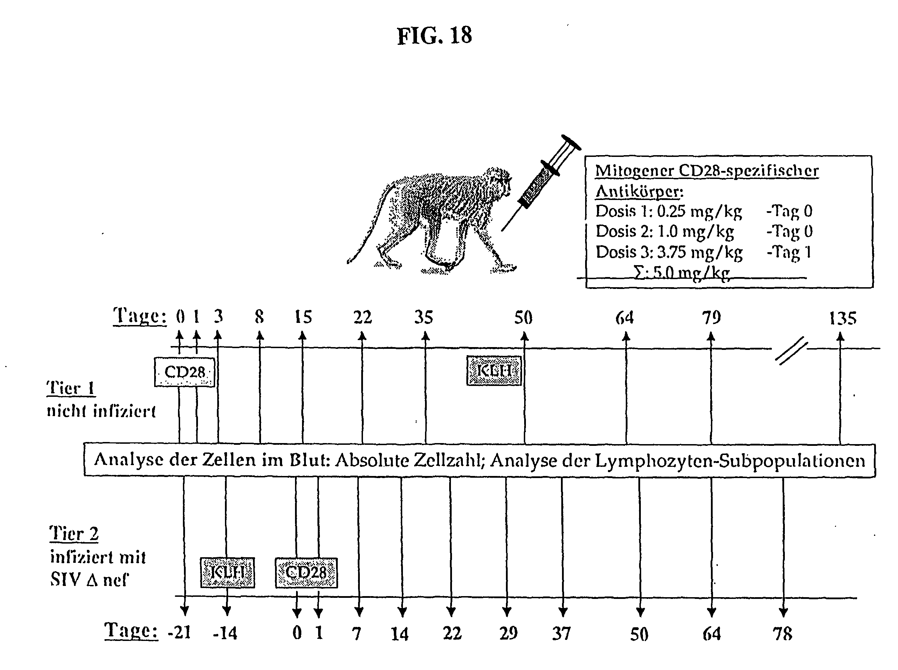

Die Figur 18 zeigt den angewandten Versuchsplan. Zwei adulte Rhesusaffen erhielten eine Dosis von 5 mg pro kg Körpergewicht eines mitogenen CD28-spezifischen mAk intravenös appliziert. Um eventuell auftretende toxische Effekte rechtzeitig erkennen zu können, wurde die Dosis in drei Einzeldosen von 0,25, 1,0 und 3,75 mg/kg Körpergewicht aufgeteilt, die im Laufe eines Tages appliziert wurden. Tier 1 war unbehandelt, Tier 2 war 22 Monate zuvor mit einem apathogenen Virus (SIVΔ^{ref}) infiziert worden. Diese Infektion spielt für die hier gezeigten Ergebnisse keine Rolle und wird nur der Vollständigkeit halber vermerkt. Tier 2 wurde außerdem 2 Wochen vor Antikörperbehandlung mit dem Modellantigen KLH immunisiert. Keines der beiden Tiere zeigte auffällige toxische Reaktionen auf die Antikörperinfusion. Zu den angezeigten Zeitpunkten wurde den Tieren Blut für die folgend beschriebenen Untersuchungen abgenommen.

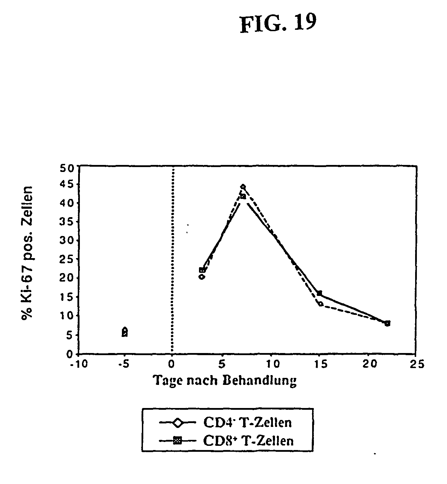

Die Fig. 19 zeigt Ki-67-Expression in T-Zellen eines CD28-stimulierten Rhesusaffen (Tier 2). Das Ki-67 Molekül wird im Zellkern von Zellen exprimiert, die sich im Zellzyklus befinden, also gerade teilungsaktiv sind. Wie die Abbildung zeigt, befanden sich vor der Behandlung etwa 5 % der CD4 und 5 % der CD8 T-Zellen im Zellzyklus. Nach der CD28 Behandlung (Tag 0) stieg die Frequenz zunächst auf 20, dann auf 45 % für beide Populationen und fiel dann wieder auf 10 % zurück. Dieses Ergebnis zeigt, daß die CD28-Stimulation in vivo die Vermehrung der CD4 und der CD8 T-Zellen stimuliert.

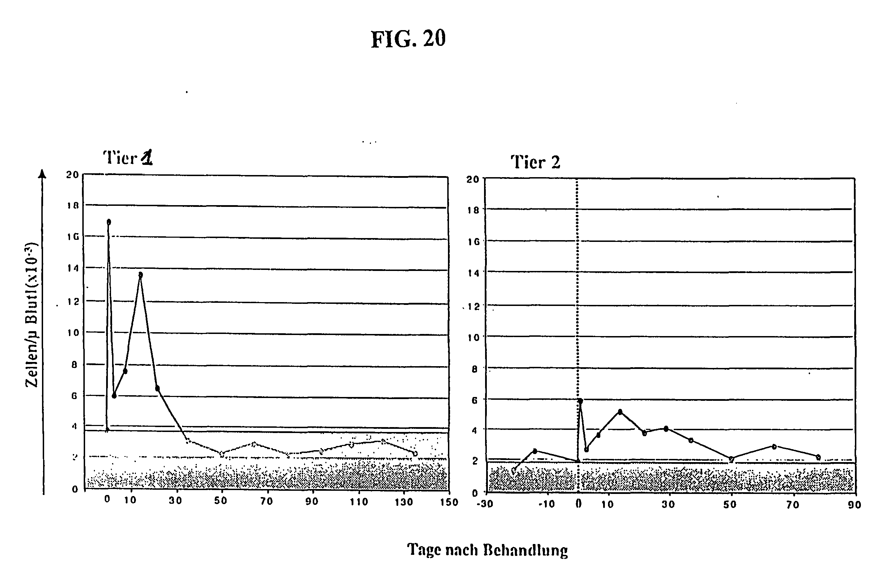

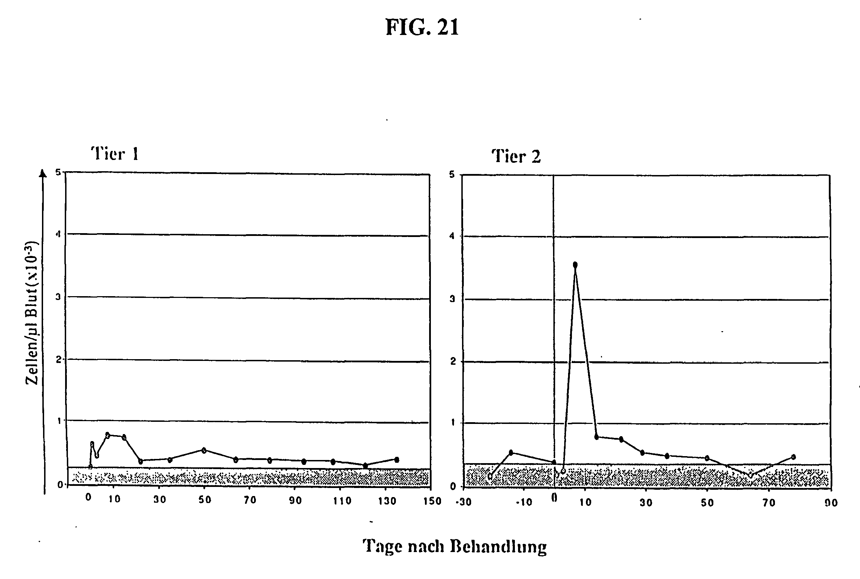

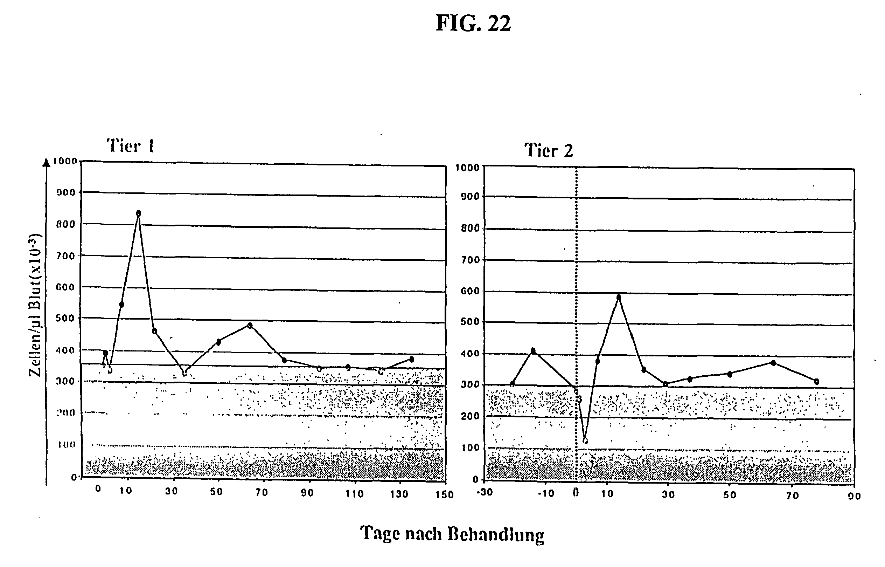

Die für die Erfindung wesentlichen Figuren 20 bis 22 zeigen schließlich die erzielbare Erhöhung der Zahl an Granulozyten, Monozyten und Thromobozyten im peripheren Blut von anti-CD28 therapierten Rhesusaffen. Die Blutbilder wurden in einem hämatologischen Labor mit einem CellDyn 4000 Gerät erhoben und sind hier als Zellen/Mikroliter Blut im Zeitverlauf dargestellt. Fig. 20 belegt die Erhöhung der Zahl an neutrophilen Granulozyten im peripheren Blut von anti-CD28 therapierten Rhesusaffen. Beide Tiere zeigten eine dramatische Erhöhung gegenüber dem Ausgangswert. Dieser wurde bei Tier 1 nach einem Monat, bei Tier 2 etwas verzögert wieder erreicht. Die Therapie könnte die gängige Behandlung mit G-CSF in der Knochenmarktransplantation überflüssig machen. Fig. 21 demonstriert die Erhöhung der Zahl an Monozyten im peripheren Blut von anti-CD28 therapierten Rhesusaffen. In beiden Tieren wurde ein deutlicher Anstieg über den Ausgangswert mit langsamer Wiederangleichung an Normalwerte während der folgenden zwei Monate beobachtet. Fig. 22 schlielich zeigt die Erhöhung der Zahl an Thrombozyten im peripheren Blut von anti-CD28 therapierten Rhesusaffen. Beide Tiere zeigten eine dramatische Erhöhung gegenüber dem Ausgangswert. Dieser wurde bei Tier 1 nach einem Monat, bei Tier 2 etwas früher wieder erreicht.

## Patentansprüche

1. Verwendung monoklonaler Antikörper, welche für CD28 spezifisch sind und T-Lymphozyten mehrerer bis aller Untergruppen ohne Besetzung eines Antigenrezeptors der T-Lymphozyten und somit antigenunspezifisch aktivieren, zur Herstellung einer pharmazeutischen Zusammensetzung zur Behandlung von Erkrankungen mit reduzierter Anzahl an Blutzellen, welche kein CD28 tragen, wobei die Erkrankung ausgewählt ist aus der Gruppe bestehend aus "Granulozytopenie, insbesondere Neutropenie und Monozytopenie, Thrombozytopenie, aplastische Anämien, Knochenmarksversagen mit Aplasie/Hypoplasie des Knochenmarks, Panzytopenie, Fanconi-Anämie, erworbenen aplastischen Anämien, insbesondere idiopatische aplastische Anämie und sekundäre aplastische Anämien sowie Anämie, Thrombozytopenie und/oder Granulozytopenie bei akuter Leukämie.

## Claims

1. The use of monoclonal antibodies that are specific of CD28 and that activate the T lymphocytes of several to all subgroups without occupying an antigen receptor of the T lymphocytes, thereby activating the T lymphocytes in an antigen-unspecific manner, for producing a pharmaceutical preparation for treating diseases comprising a reduced number of blood cells that lack CD28, wherein the disease is selected from the group comprising "granulocytopenia, in particular neutropenia and monocytopenia, thrombocytopenia, aplastic anemias, bone marrow failure with aplasia/hypoplasia of the bone marrow, pancytopenia, Fanconi anemia, acquired aplastic anemias, in particular idiopathic aplastic anemia and secondary aplastic anemias, and anemia, thrombocytopenia and/or granulocytopenia with acute leukemia".

## Revendications

1. Utilisation d'anticorps monoclonaux, qui sont spécifiques de CD28 et qui activent les lymphocytes T de plusieurs à tous les sous-groupes sans occupation d'un récepteur antigénique des lymphocytes T et donc de manière non-spécifique de l'antigène, pour la production d'une préparation pharmaceutique pour le traitement de maladies avec un nombre réduit de cellules de sang qui ne portent pas de CD28, dans laquelle la maladie est choisie à partir du groupe comprenant "la granulocytopénie, en particulier la neutropénie et la monocytopénie, la thrombocytopénie, les anémies aplastique, l'insuffisance médullaire avec aplasie/hypoplasie de la moelle osseuse, la pancytopénie, l'anémie de Fanconi, les anémies aplastiques acquises, en particulier l'anémie aplastique idiopathique et les anémies aplastiques secondaires, et l'anémie, la thrombocytopénie et/ou la granulocytopénie avec leucémie aiguë".
